# EUROPÄISCHE PATENTSCHRIFT

(11) **EP 1 161 225 B1**
(45) Veröffentlichungstag und Bekanntmachung des Hinweises auf die Patenterteilung: **06.10.2004**
(21) Anmeldenummer: 00906289.4
(22) Anmeldetag: 07.02.2000
(51) Int. Cl.: A61K 9/12, A61P 11/06

(54) **DOSIERAEROSOLE MIT ISOBUTAN ALS TREIBMITTEL**
METERED DOSE INHALERS WITH ISOBUTANE AS PROPELLANT
AEROSOLS-DOSEURS CONTENANT DE L'ISOBUTANE COMME AGENT PROPULSEUR

(30) Priorität: 12.03.1999 DE 19911064
(43) Veröffentlichungstag der Anmeldung: 12.12.2001
(73) Patentinhaber: IG Sprühtechnik GmbH & Co. Kg, 79664 Wehr (DE)
(72) Erfinder: GUCK, Franz, D-79618 Rheinfelden (DE); WARNKE, Gieselher, D-79737 Herrischried (DE)
(74) Vertreter: Flaccus, Rolf-Dieter, Dr.
(86) Internationale Anmeldenummer: PCT/EP2000/000946
(87) Internationale Veröffentlichungsnummer: WO 2000/054748

(56) Entgegenhaltungen:
- EP-A- 0 504 112
- WO-A-93/04671
- DE-A- 4 132 176
- US-A- 5 202 110
- US-A- 5 292 499

## Beschreibung

Gegenstand der vorliegenden Erfindung sind Dosieraerosole mit antiasthmatischen Wirkstoffen und druckverflüssigtem Isobutan oder dessen Gemisch mit oberflächenaktiven Mitteln als Treibmittel sowie die Verwendung dieser Dosieraerosole zur Behandlung allergischer Erkrankungen bei Mensch und Tier.

Seit Jahrzehnten werden Aerosol-Druckgaspackungen, kurz Dosieraerosole genannt, unter Verwendung druckverflüssigter Gase oder auch komprimierter Gase als Treibnittel produziert und verwendet. Solche Dosieraerosole bestehen im allgemeinen aus einem Druckbehälter, vorzugsweise aus Metall oder Glas mit einer Ventilkonstruktion zur Entnahme des Inhalts und dem eigentlichen, zu versprühenden Mittel. Dieses Mittel, auch Wirkstofflösung genannt, kann vielfältiger Natur sein. In den meisten Fällen besteht der Behälterinhalt aus dem zu versprühenden Wirkstoff und einem Treibmittel in Form eines druckverflüssigten Gases. Dieses Gas, eingesetzt werden auch Gasgemische, sollte im Idealfall in jedem Verhältnis mit dem Wirkstoff mischbar sein, so daß eine einzige flüssige Phase entsteht oder es sollte mit dem Wirkstoff eine gut aufzuschüttelnde Suspension bilden, über der sich eine Gasphase bildet. Je nach enthaltenem Mittel werden diese Dosieraerosole im kosmetischen und medizinischen Pereich aber auch als Raumspray, Insektizidspray und ähnliches eingesetzt.

Die Treibmittel solcher Aerosole haben besondere Anforderungen zu erfüllen. Sie dürfen auf keinen Fall mit dem Mittel eine Reaktion eingehen. Sie müssen nicht irritierend und nicht toxisch sein. Als besonders geeignet haben sich daher die Fluorchlorkohlenwasserstoffe, kurz FCKW genannt, herausgestellt. Bei diesen Stoffen handelt es sich um gasförmige oder flüssige chemische Stoffe mit besonders günstigen Produkteigenschaften. Sie sind chemisch sehr stabil, unbrennbar und für Mensch und Tier ungiftig. Für antiasthmatische Dosieraerosole werden üblicherweise die Typen R 11, R114 sowie R 12 verwendet. Nachteilig ist ihre ozonabbauende Wirkung, die es aufgrund internationaler Verträge erforderlich macht, die Produktion und die Verwendung dieser Stoffe langfristig ganz einzustellen. Ersatz- und Alternativlösungen sind daher notwendig geworden. Diese Alternativmittel müssen qualitativ den FCKW's vergleichbar sein, insbesondere gesundheitlich unbedenklich und zudem ökologisch verträglich sein. Doch auch die als Ersatzstoffe vielfach propagierten teilhalogenierten FCKW's, sogenannte H-FCKW's und H-FKW's erfüllen nicht diese Anforderungen. Ihre ökologische Verträglichkeit ist zwar besser, doch auch sie weisen immer noch eine Ozonabbaufähigkeit von etwa 5% auf. Hinzu kommt aber noch ein für die Verwendung als Treibmittel für die genannten Zwecke besonderer Nachteil. Zur besseren Dispergierung eines Wirkstoffes im Treibmittel müssen in einigen Fällen, insbesondere bei der Verwendung von antiasthmatischen Wirkstoffen, sogenannte oberflächenaktive Mittel eingesetzt werden. Diese Mittel lösen sich zwar in den FCKW's, nicht aber in den Alternativtreibmitteln des H-FKW-Typs auf, so daß hier stets der Zusatz eines polaren Lösungsmittels erforderlich ist.

In der DE 41 32 176 werden Dosieraerosole mit Isobutan als Treibmittel beschrieben. Das druckverflüssigte Isobutan ist ökologisch verträglich und in der Lage, oberflächenaktive Stoffe, die üblicherweise beispielsweise antiasthmatischen Dosieraerosolen beigefügt werden, ohne Zusatz eines weiteren Lösungsmittels zu lösen. Damit erfüllt Isobutan die oben genannten Anforderungen. Die in der DE 41 32 176 verwendeten Wirkstoffe gehören - mit Ausnahme des DNCG, das zu den nichtsteroidalen Entzündungshemmern zählt - der Stoffklasse der Isoprenalin-Abkömmlinge, den sogenannten β-Sympathomimetika, an. Dosieraerosole mit steroidalen Wirkstoffen und Isobutan als Treibmittel sind dagegen noch nicht bekannt.

In WO93/04671 wird darüberhinaus eine Aerosolzusammensetzung beschrieben, die als Treibmittel n-Butan oder Di methylether oder eine Kombination beider Substanzen enthält. Als oberflächenaktive Substanzen finden Glycerolphosphatide Verwendung. Als weitere Hilfsstoffe können auch zusätzliche Treibmittel eingesetzt werden, wie z.B. Propan oder Isobutan.

Eine allgemein gehaltene Anleitung zur Herstellung medizinischer Aerosole, bestehend aus einem Treibmittel, einer oberflächenaktiven Substanz und einem pharmazeutischen Wirkstoff, wird in der US 52 92 499 gegeben. Dabei werden oberflächenaktive Substanzen mit dem Wirkstoff, dem Treibmittel und Wasser in einem Verhältnis gemischt, in dem sich Umkehrmizellen, den Wirkstoff beinhaltend, im Treibmittel bilden können.

Aufgabe der vorliegenden Erfindung war es daher, Dosieraerosole bereitzustellen, die neben einem Wirkstoff aus der Stoffklasse der Glucocorticoide ein Treibmittel enthalten, das neben seiner ökologischen Verträglichkeit auch in der Lage sein sollte, oberflächenaktive Stoffe, die üblicherweise beispielsweise antiasthmatischen Dosieraerosolen beigefügt werden, ohne Zusatz eines weiteren Lösungsmittels zu lösen.

Überraschenderweise wurde diese Aufgabe mit Isobutan als Treibmittel, oberflächenaktiver Substanz und einem Wirkstoff entsprechend den kennzeichnenden Merkmalen von Anspruch 1 gelöst.

Die oberflächenaktiven Mittel sind dabei ausgewählt aus den Estern oder Teilestern der Fettsäuren von 6 bis 22 C-Atomen mit einem aliphatischen mehrwertigen Alkohol oder cyclischen Anhydriden dieser Alkohole oder Polyoxyethylen- oder Polyoxypropylenderivaten dieser Ester.

Auch die Ölsäure selbst ist im Sinne der vorliegenden Erfindung als oberflächenaktives Mittel einsetzbar.

Bevorzugte oberflächenaktive Mittel sind neben der Ölsäure die Oleate von Sorbitan, insbesondere Span 85.

Als Wirkstoffe für die erfindungsgemäßen Dosieraerosole kommen die an sich bekannten Glucocorticoide in Frage, wobei insbesondere Cortisol, Prednison, Prednisolon, Methylprednisolon, Triamcinolon, Prednilyden, Fluocortolon, Paramethason, Dexamethason, Betamethason, Beclometason, Budesonid und/oder deren antiasthmatisch wirksame Derivate zu nennen sind. Auch Mischungen dieser Wirkstoffe sind einsetzbar. Bevorzugt sind erfindungsgemäß die Glucocorticoide, die sich bereits als Antiasthmatika bewährt haben, beispielsweise Beclometasonderivat und/oder Budesonid.

Geeignete Treibmittelmischungen setzen sich zusammen aus 0,05 bis 5,88 Gew.-%, vorzugsweise 0,5 bis 2,0 Gew.-% des nicht ionischen oberflächenaktiven Mittels und 94,12 bis 99,95 Gew.-%, vorzugsweise 97,0 bis 99,4 Gew.-% Isobutan. Bei Verwendung des besonders geeigneten, nicht ionischen oberflächenaktiven Mittels Span 85 zur Herstellung der erfindungsgemäßen Dosieraerosole haben sich folgende Rezepturbilder als vorteilhaft herausgestellt:

| | | |
|---|---|---|
| Rezepturbild 1 | Glucocorticoid | 1,00 % - 10,00 % |
| | Span 85 | 0,50 % - 5, 00 % |
| | Isobutan | 98,50 % - 85, 00 % |
| Rezepturbild 2 | Glucocorticoid I | 1,00 % - 10,00 % |
| | Glucocorticoid II | 0,50 % - 5,00 % |
| | Span 85 | 0,50 % - 5,00 % |
| | Isobutan | 98,00 % - 80,00 % |
| Rezepturbild 3 | Glucocorticoid | 0,20 % - 1,00 % |
| | Span 85 | 0,05 % - 0,50 % |
| | Isobutan | 99,75 % - 98,50 % |
| Rezepturbild 4 | Glucocorticoid | 0,50 % - 3,00 % |
| | Span 85 | 0,50 % - 5,00 % |
| | Isobutan | 99,00 % - 92,00 % |

Bevorzugt sind die folgenden Aerosolmischungen, bei denen 1 Aerosoldose die nachfolgenden Mengen enthält:

| | | | |
|---|---|---|---|
| Dosieraerosol 1 | Glucocorticoid | 3,35 % | (200 mg) |
| | Span 85 | 2,35 % | (140 mg) |
| | Isobutan | 94,30 % | (5630 mg) |
| Dosieraerosol 2 | Glucocorticoid I | 3,35 % | (200 mg) |
| | Glucocorticoid II | 1,66 % | (100 mg) |
| | Span 85 | 3,52 % | (210 mg) |
| | Isobutan | 91,46 % | (5460 mg) |
| Dosieraerosol 3 | Glucocorticoid | 0,71 % | (60 mg) |
| | Span 85 | 0,18 % | (15 mg) |
| | Isobutan | 99,11 % | (8395 mg) |
| Dosieraerosol 4 | Glucocorticoid | 1,66 % | (100 mg) |
| | Span 85 | 2,32 % | (140 mg) |
| | Isobutan | 96,03 % | (5800 mg) |

Bei der Verwendung von Ölsäure als oberflächenaktives Mittel werden bevorzugt 0,01 bis 0,11 Gew.-% eingesetzt. Als vorteilhaft hat sich bei Verwendung der Ölsäure das folgende Rezepturbild herausgestellt:

| | | |
|---|---|---|
| Rezepturbild 5 | Glucocorticoid | 0,10 % - 0,50 % |
| | Ölsäure | 0,01 % - 0,10% |
| | Isobutan | 99,89 % - 99,40 % |

Bevorzugt ist dabei eine Aerosolmischung, bei der 1 Aerosoldose die nachfolgenden Mengen enthält:

| | | |
|---|---|---|
| Dosieraerosol 5 | Glucocorticoid | 0,35 % (30 mg) |
| | Ölsäure | 0,03 % (3 mg) |
| | Isobutan | 99,62 % (8550 mg) |

Als besonders geeigneter Wirkstoff hat sich erfindungsgemäß Budesonid herausgestellt. Als oberflächenaktives Mittel wird vorzugsweise Span 85 eingesetzt. Dabei hat sich das folgende Rezepturbild als vorteilhaft herausgestellt:

| | | |
|---|---|---|
| Rezepturbild 6 | Budesonid | 0,10 % - 1,00 % |
| | Span 85 | 0,50 % - 2,00 % |
| | Isobutan | 97,00 % - 99,40 % |

Ein Budesonid-Dosieraerosol mit 0,2 mg Wirkstoff/Hub enthält beispielsweise pro Aerosoldose:

| | | |
|---|---|---|
| Dosieraerosol 6 | Budesonid | 0,70 % (40 mg) |
| | Span 85 | 1,23 % (70 mg) |
| | Isobutan | 98,07 % (5590 mg) |

Ein Budesonid-Dosieraerosol mit 0,05 mg Wirkstoff/Hub enthält beispielsweise pro Aerosoldose:

| | | |
|---|---|---|
| Dosieraerosol 7 | Budesonid | 0,18 % (10 mg) |
| | Span 85 | 1,22 % (70 mg) |
| | Isobutan | 98,60 % (5620 mg) |

Die erfindungsgemäßen Aerosole können durch Mischen der verschiedenen Bestandteile unter Bedingungen, bei denen das Treibmittel und das oberflächenaktive Mittel flüssig und der Wirkstoff in fester Phase vorliegt, hergestellt werden.

Die Wirkstoffsuspension wird durch das Ventil unter Druck in die verclinchte Dose, die zu Beginn des Füllvorganges Raumtemperatur hat, gefüllt. Die Suspension hat eine Temperatur von ca. -8 bis -10 °C. Anschließend wird mit dem Treibmittel nachgefüllt und so das Ventil gleichzeitig gereinigt.

Die erfindungsgemäßen Dosieraerosole können bei der Behandlung von Mensch und Tier, insbesondere bei der Behandlung allergischer Erkrankungen der Luftwege wie Asthma oder allergischer Rhinitis (Heuschnupfen), vorzugsweise durch orale oder nasale Inhalation, eingesetzt werden.

## Patentansprüche

1. Dosieraerosol auf der Basis einer Suspension bestehend aus antiasthmatischem Wirkstoff, ausgewählt aus der Reihe der Glucocorticoide oder deren Derivaten, druckverflüssigtem Isobutan als ausschließlichem Treibmittel sowie in druckverflüssigtem Isobutan löslichen oberflächenaktiven Substanzen, ausgewählt aus der Gruppe umfassend Olsäure die Ester oder Teilester der Fettsäuren von 6 bis 22 C-Atomen mit einem aliphatischen mehrwertigen Alkohol oder cyclischen Anhydriden dieser Alkohole und Polyoxyethylen- oder Polyoxypropylenderivaten dieser Ester.

2. Dosieraerosol gemäß Anspruch 1, **dadurch gekennzeichnet, dass** der antiasthmatische Wirkstoff durch Cortisol, Prednison, Prednisolon, Methylprednisolon, Triamcinolon, Prednilyden, Fluocortolon, Paramethason, Dexamethason, Betamethason, Beclometason, Budesonid und/oder deren antiasthmatisch wirksame Derivate und/oder deren Mischungen gebildet wird.

3. Dosieraerosol nach Anspruch 1 oder 2, **dadurch gekennzeichnet, dass** die oberflächenaktive Substanz durch Ölsäure oder Oleate von Sorbitan, insbesondere Span 85, gebildet wird.

4. Dosieraerosol gemäß Anspruch 1 oder 2, **dadurch gekennzeichnet, dass** es dem Rezepturbild
| | |
|---|---|
| Glucocorticoid | 1,00 % - 10,00 % |
| Span 85 | 0,50 % - 5,00 % |
| Isobutan | 98,50 % - 85,00 % |
entspricht.

5. Dosieraerosol gemäß Anspruch 1 oder 2, **dadurch gekennzeichnet, dass** es dem Rezepturbild
| | |
|---|---|
| Glucocorticoid I | 1,00 % - 10,00 % |
| Glucocorticoid II | 0,50 % - 5,00 % |
| Span 85 | 0,50 % - 5,00 % |
| Isobutan | 98,00 % - 80,00 % |
entspricht.

6. Dosieraerosol gemäß Anspruch 1 oder 2, **dadurch gekennzeichnet, dass** es dem Rezepturbild
| | |
|---|---|
| Glucocorticoid | 0,20 % - 1,00 % |
| Span 85 | 0,05 % - 0,50 % |
| Isobutan | 99,75 % - 98,50 % |
entspricht.

7. Dosieraerosol gemäß Anspruch 1 oder 2, **dadurch gekennzeichnet, dass** es dem Rezepturbild
| | |
|---|---|
| Glucocorticoid | 0,50 % - 3,00 % |
| Span 85 | 0,50 % - 5,00 % |
| Isobutan | 99,00 % - 92,00 % |
entspricht.

8. Dosieraerosol gemäß Anspruch 1 oder 2, **dadurch gekennzeichnet, dass** es aus
| | |
|---|---|
| Glucocorticoid | 3,35 % |
| Span 85 | 2,35 % |
| Isobutan | 94,30 % |
besteht.

9. Dosieraerosol gemäß Anspruch 1 oder 2, **dadurch gekennzeichnet, dass** es aus
| | |
|---|---|
| Glucocorticoid I | 3,35 % |
| Glucocorticoid II | 1,66 % |
| Span 85 | 3,52 % |
| Isobutan | 91,46 % |
besteht.

10. Dosieraerosol gemäß Anspruch 1 oder 2, **dadurch gekennzeichnet, dass** es aus
| | |
|---|---|
| Glucocorticoid | 0,71 % |
| Span 85 | 0,18 % |
| Isobutan | 99,11 % |
besteht.

11. Dosieraerosol gemäß Anspruch 1 oder 2, **dadurch gekennzeichnet, dass** es aus
| | |
|---|---|
| Glucocorticoid | 1,66 % |
| Span 85 | 2,32 % |
| Isobutan | 96,03 % |
besteht.

12. Dosieraerosol gemäß Anspruch 1 oder 2, **dadurch gekennzeichnet, dass** es dem Rezepturbild
| | |
|---|---|
| Glucocorticoid | 0,10 % - 0,50 % |
| Ölsäure | 0,01 % - 0,10 % |
| Isobutan | 99,89 % - 99,40 % |
entspricht.

13. Dosieraerosol gemäß Anspruch 1 oder 2, **dadurch gekennzeichnet, dass** es aus
| | |
|---|---|
| Glucocorticoid | 0,35 % |
| Ölsäure | 0,03 % |
| Isobutan | 99,62 % |
besteht.

14. Dosieraerosol gemäß Anspruch 1 oder 2, **dadurch gekennzeichnet, dass** es dem Rezepturbild
| | |
|---|---|
| Budesonid | 0,10 % - 1,00 % |
| Span 85 | 0,50 % - 2,00 % |
| Isobutan | 97,00 % - 99,40 % |
entspricht.

15. Dosieraerosol gemäß Anspruch 1 oder 2, **dadurch gekennzeichnet, dass** es aus
| | |
|---|---|
| Budesonid | 0,70 % |
| Span 85 | 1,23 % |
| Isobutan | 98,07 % |
besteht.

16. Dosieraerosol gemäß Anspruch 1 oder 2, **dadurch gekennzeichnet, dass** es aus
| | |
|---|---|
| Budesonid | 0,18 % |
| Span 85 | 1,22 % |
| Isobutan | 98,60 % |
besteht.

17. Verfahren zur Herstellung von Dosieraerosolen nach einem der vorangehenden Ansprüche, **dadurch gekennzeichnet, dass** man Treibmittel und oberflächenaktives Mittel in flüssiger Form und den Wirkstoff als Feststoff miteinander vermischt und die flüssige Suspension durch das Ventil unter Druck in die zu Beginn des Füllvorgangs Raumtemperatur aufweisende Sprühdose einfüllt und schließlich unter Nachfüllen von Treibmittel das Ventil der Sprühdose reinigt.

## Claims

1. Controlled dosage aerosol on the basis of a suspension consisting of anti-asthmatic active substance selected from the family of the glucocorticoids or their derivatives, pressure-liquefied isobutane as exclusive propellant, as well as surface-active agents soluble in pressure-liquefied isobutane and selected from the group comprising oleic acid, esters or partial esters of fatty acids of 6 to 22 C atoms with an aliphatic polyhydric alcohol or cyclic anhydrides of said alcohols and polyoxyethylene derivatives or polyoxypropylene derivatives of said esters.

2. Controlled dosage aerosol according to claim 1, **characterized in that** the anti-asthmatic active agent is constituted by cortisol, prednisone, prednisolone, methylprednisolone, triamcinolone, prednilydene, fluocortolone, paramethasone, dexamethasone, betamethasone, beclomethasone, budesonide and/or their anti-asthmatically active derivatives and/or mixtures thereof.

3. Controlled dosage aerosol according to claim 1 or 2, **characterized in that** the surface-active agent is constituted by oleic acid or oleates of sorbitan, especially Span 85.

4. Controlled dosage aerosol according to claim 1 or 2, **characterized in that** it corresponds to the formulation:
| | |
|---|---|
| Glucocorticoid | 1.00% - 10.00% |
| Span 85 | 0.50% - 5.00% |
| Isobutane | 98.50% - 85.00% |

5. Controlled dosage aerosol according to claim 1 or 2, **characterized in that** it corresponds to the formulation:
| | |
|---|---|
| Glucocorticoid I | 1.00% - 10.00% |
| Glucocorticoid II | 0.50% - 5.00% |
| Span 85 | 0.50% - 5.00% |
| Isobutane | 98.00% - 80.00% |

6. Controlled dosage aerosol according to claim 1 or 2, **characterized in that** it corresponds to the formulation:
| | |
|---|---|
| Glucocorticoid | 0.20% - 1.00% |
| Span 85 | 0.05% - 0.50% |
| Isobutane | 99.75% - 98.50% |

7. Controlled dosage aerosol according to claim 1 or 2, **characterized in that** it corresponds to the formulation:
| | |
|---|---|
| Glucocorticoid | 0.50% - 3.00% |
| Span 85 | 0.50% - 5.00% |
| Isobutane | 99.00% - 92.00% |

8. Controlled dosage aerosol according to claim 1 or 2, **characterized in that** it consists of:
| | |
|---|---|
| Glucocorticoid | 3.35% |
| Span 85 | 2.35% |
| Isobutane | 94.30% |

9. Controlled dosage aerosol according to claim 1 or 2, **characterized in that** it consists of:
| | |
|---|---|
| Glucocorticoid I | 3.35% |
| Glucocorticoid II | 1.66% |
| Span 85 | 3.52% |
| Isobutane | 91.46% |

10. Controlled dosage aerosol according to claim 1 or 2, **characterized in that** it consists of:
| | |
|---|---|
| Glucocorticoid | 0.71% |
| Span 85 | 0.18% |
| Isobutane | 99.11% |

11. Controlled dosage aerosol according to claim 1 or 2, **characterized in that** it consists of:
| | |
|---|---|
| Glucocorticoid | 1.66% |
| Span 85 | 2.32% |
| Isobutane | 96.03% |

12. Controlled dosage aerosol according to claim 1 or 2, **characterized in that** it corresponds to the formulation:
| | |
|---|---|
| Glucocorticoid | 0.10% - 0.50% |
| Oleic acid | 0.01% - 0.10% |
| Isobutane | 99.89% - 99.40% |

13. Controlled dosage aerosol according to claim 1 or 2, **characterized in that** it consists of:
| | |
|---|---|
| Glucocorticoid | 0.35% |
| Oleic acid | 0.03% |
| Isobutane | 99.62% |

14. Controlled dosage aerosol according to claim 1 or 2, **characterized in that** it corresponds to the formulation:
| | |
|---|---|
| Budesonide | 0.10% - 1.00% |
| Span 85 | 0.50% - 2.00% |
| Isobutane | 97.00% - 99.40% |

15. Controlled dosage aerosol according to claim 1 or 2, **characterized in that** it consists of:
| | |
|---|---|
| Budesonide | 0.70% |
| Span 85 | 1.23% |
| Isobutane | 98.07% |

16. Controlled dosage aerosol according to claim 1 or 2, **characterized in that** it consists of
| | |
|---|---|
| Budesonide | 0.18% |
| Span 85 | 1.22% |
| Isobutane | 98.60% |

17. Process for the production of controlled dosage aerosols according to any one of the preceding claims, **characterized in that** propellant and surface-active agent, in liquid form, and the active substance, as a solid, are mixed with one another, and the liquid suspension is filled through the valve into the spray tin under pressure, which spray tin at the start of the filling procedure has room temperature, and that finally the valve of the spray tin is cleaned by filling up with propellant.

## Revendications

1. Aérosol de dosage à base d'une suspension constituée d'une substance active anti-asthmatique, choisie dans la série des glucocorticoïdes ou leurs dérivés, d'isobutane liquéfié sous pression comme agent propulseur exclusif ainsi que de substances tensioactives solubles dans l'isobutane liquéfié sous pression, choisies dans le groupe comprenant l'acide oléique, les esters ou esters partiels des acides gras comprenant 6 à 22 atomes de carbone avec un alcool aliphatique polyvalent ou les anhydrides cycliques de ces alcools et les dérivés polyoxyéthylène ou polyoxypropylène de ces esters.

2. Aérosol de dosage selon la revendication 1, **caractérisé en ce que** la substance active anti-asthmatique est formée par le cortisol, le prednisone, le prednisolone, le méthylprednisolone, le triamcinolone, le prednilydène, le fluocortolone, le paraméthasone, le dexaméthasone, le bêtaméthasone, le béclometasone, le budésonide et/ou leurs dérivés actifs en tant qu'antiasthmatiques et/ou leurs mélanges.

3. Aérosol de dosage selon la revendication 1 ou 2, **caractérisé en ce que** la substance tensioactive est formée par l'acide oléique ou les oléates de sorbitane, en particulier le Span 85.

4. Aérosol de dosage selon la revendication 1 ou 2, **caractérisé en ce qu'**il correspond à la formulation :
| | |
|---|---|
| Glucocorticoïde | 1,00% - 10,00% |
| Span 85 | 0,50% - 5,00% |
| Isobutane | 98,50% - 85,00%. |

5. Aérosol de dosage selon la revendication 1 ou 2, **caractérisé en ce qu'**il correspond à la formulation :
| | |
|---|---|
| Glucocorticoïde 1 | 1,00% - 10,00% |
| Glucocorticoïde II | 0,50% - 5,00% |
| Span 85 | 0,50% - 5,00% |
| Isobutane | 98,00% - 80,00%. |

6. Aérosol de dosage selon la revendication 1 ou 2, **caractérisé en ce qu'**il correspond à la formulation :
| | |
|---|---|
| Glucocorticoïde | 0,20% - 1,00% |
| Span 85 | 0,05% - 0,50% |
| Isobutane | 99,75% - 98,50%. |

7. Aérosol de dosage selon la revendication 1 ou 2, **caractérisé en ce qu'**il correspond à la formulation :
| | |
|---|---|
| Glucocorticoïde | 0,50% - 3,00% |
| Span 85 | 0,50% - 5,00% |
| Isobutane | 99,00% - 92,00%. |

8. Aérosol de dosage selon la revendication 1 ou 2, **caractérisé en ce qu'**il est constitué de :
| | |
|---|---|
| Glucocorticoïde | 3,35% |
| Span 85 | 2,35% |
| Isobutane | 94,30%. |

9. Aérosol de dosage selon la revendication 1 ou 2, **caractérisé en ce qu'**il est constitué de :
| | |
|---|---|
| Glucocorticoïde 1 | 3,35% |
| Glucocorticoïde II | 1,66% |
| Span 85 | 3,52% |
| Isobutane | 91,46%. |

10. Aérosol de dosage selon la revendication 1 ou 2, **caractérisé en ce qu'**il est constitué de :
| | |
|---|---|
| Glucocorticoïde | 0,71 % |
| Span 85 | 0,18% |
| Isobutane | 99,11%. |

11. Aérosol de dosage selon la revendication 1 ou 2, **caractérisé en ce qu'**il est constitué de :
| | |
|---|---|
| Glucocorticoïde | 1,66% |
| Span 85 | 2,32% |
| Isobutane | 96,03%. |

12. Aérosol de dosage selon la revendication 1 ou 2, **caractérisé en ce qu'**il correspond à la formulation :
| | |
|---|---|
| Glucocorticoïde | 0,10% - 0,50% |
| Acide oléique | 0,01% - 0,10% |
| Isobutane | 99,89% - 99,40%. |

13. Aérosol de dosage selon la revendication 1 ou 2, **caractérisé en ce qu'**il est constitué de :
| | |
|---|---|
| Glucocorticoïde | 0,35% |
| Acide oléique | 0,03% |
| Isobutane | 99,62%. |

14. Aérosol de dosage selon la revendication 1 ou 2, **caractérisé en ce qu'**il correspond à la formulation :
| | |
|---|---|
| Budésonide | 0,10% - 1,00% |
| Span 85 | 0,50% - 2,00% |
| Isobutane | 97,00% - 99,40%. |

15. Aérosol de dosage selon la revendication 1 ou 2, **caractérisé en ce qu'**il est constitué de :
| | |
|---|---|
| Budésonide | 0,70% |
| Span 85 | 1,23% |
| Isobutane | 98,07%. |

16. Aérosol de dosage selon la revendication 1 ou 2, **caractérisé en ce qu'**il est constitué de :
| | |
|---|---|
| Budésonide | 0,18% |
| Span 85 | 1,22% |
| Isobutane | 98,60%. |

17. Procédé pour préparer des aérosols de dosage selon l'une quelconque des revendications précédentes, **caractérisé en ce qu'**on mélange l'un avec l'autre l'agent propulseur et l'agent tensioactif sous forme liquide et la substance active sous forme solide et on remplit la suspension liquide via la soupape sous pression dans la bombe d'aérosol se trouvant à température ambiante au début du processus de remplissage et on nettoie enfin la soupape de la bombe d'aérosol par remplissage ultérieur d'agent propulseur.
